# EUROPEAN PATENT SPECIFICATION

(11) **EP 3 640 235 B1**
(45) Date of publication and mention of the grant of the patent: **30.03.2022**
(21) Application number: 18201149.4
(22) Date of filing: 18.10.2018
(51) Int. Cl.: C07C 45/46, C07C 49/665

(54) **PROCESS FOR THE PREPARATION OF CYCLIC PERI-SUCCINOYLACENAPHTHENE AND RELATED 1,4-DIKETO COMPOUNDS**
VERFAHREN ZUR HERSTELLUNG VON CYCLISCHEM PERI-SUCCINOYLACENAPHTHEN UND ZUGEHÖRIGE 1,4-DIKETO-VERBINDUNGEN
PROCÉDÉ DE PRÉPARATION DE COMPOSÉS PERI-SUCCINOYLACENAPHTHENES CYCLIQUES ET DE COMPOSÉS 1,4-DICÉTONE ASSOCIÉS

(43) Date of publication of application: 22.04.2020
(73) Proprietor: Clariant Plastics & Coatings Ltd, 4132 Muttenz (CH)
(72) Inventor: AWARE, Umesh Ramdas, Nasik - 422101 (IN); SAIT, Mohamed M., Bangalore 562107 (IN)
(74) Representative: Rippel, Hans Christoph

(56) References cited:
- CH-A- 131 959
- DE-C- 542 618
- FR-A- 636 065
- LOUIS F. FIESER ET AL: "CONDENSATIONS AND RING CLOSURES IN THE NAPHTHALENE SERIES. III. 1 PERI-SUCCINOYLACENAPHTHENE", JOURNAL OF THE AMERICAN CHEMICAL SOCIETY, vol. 54, no. 11, November 1932 (1932-11), pages 4347-4356, XP055529400, ISSN: 0002-7863, DOI: 10.1021/ja01350a030
- M.P. CAVA ET AL: "Pleiadene systems-I", TETRAHEDRON, vol. 21, no. 11, January 1965 (1965-01), pages 3051-3057, XP055529458, AMSTERDAM, NL ISSN: 0040-4020, DOI: 10.1016/S0040-4020(01)96924-8
- BRYAN WILSON. ROBERTS ET AL: "Evidence on the scope and limitations of ring-forming reactions of tricarbonyliron complexes of functional 1,2-disubstituted cyclobutadienes. Synthesis of tricarbonyliron complexes of cyclobutadienocycloheptatrienyl derivatives", JOURNAL OF THE AMERICAN CHEMICAL SOCIETY, vol. 92, no. 21, October 1970 (1970-10), pages 6382-6384, XP055529437, ISSN: 0002-7863, DOI: 10.1021/ja00724a071

## Description

### FIELD OF THE INVENTION

The present invention relates to a particularly advantageous simplified process for the synthesis of cyclic peri-succinoylacenaphthene and related 1,4-diketo compounds, especially 3,4,8,9- tetrahydrocyclohepta[fg]acenaphthylene-7,10-dione in high boiling aprotic non-reactive solvent. The invention further relates to their uses especially for synthesis of various pigments, dyes for high end applications, and also various intermediates for drugs or pharmaceutically active composition/ intermediates.

### BACKGROUND OF THE INVENTION

The patent document DE376635C (1923) describes a method of preparing keto-butyric acids consisting of condensing hydrocarbons and their derivatives with aluminum chloride and succinic anhydride in presence of nitrobenzene. However, it has not disclosed cyclic keto compound based on acenaphthene or derivatives.

The patent document FR636065A (1928) relates to the invention wherein with the aid of an acidic condensing agent, dicarboxylic anhydrides, such as maleic anhydride, on hydrocarbons, their derivatives or substitution products having the peri-free position, a new condensation product with the characteristics of cyclic ketones are obtained. Here, the reaction caused by the condensation takes place in two stages. Firstly, as an intermediate phase, a keto-carboxylic acid with an open chain which, by subsequent condensation, is converted into a cyclic ketone. Here the keto-carboxylic acid is isolated at first and then subjected to next step reaction for converting to a cyclic ketone compound.

The Swiss patent document CH131959A (1929) describes a process for the preparation of a cyclic compound, characterized in that naphthalene and succinic anhydride are condensed with the aid of aluminum chloride. Here again, the keto-carboxylic acid is isolated at first and then a cyclic keto compound was formed by using AlCl3_{/}NaCl melt. Also, alternative route using solvents and AlCl₃ is described where the intermediates were isolated and purified.

The patent document US1759111 (1930) described synthesis of 3,4,8,9-tetrahydrocyclohepta[fg]acenaphthylene-7,10-dione using acenaphthene, succinic anhydride and sodium melt (AlCl₃ + NaCl). Also, alternative route using solvents such as nitro benzene and AlCl₃ is described where the intermediates were isolated and purified. The final product 3,4,8,9-tetrahydrocyclohepta[fg]acenaphthylene-7,10-dione is obtained by cyclization using eutectic melt of sodium-aluminum chloride.

Fieser et al (J. Am. Chem. Soc. 1932, Nov., p.4347 - 4356) described synthesis of 3,4,8,9- tetrahydrocyclohepta[fg]acenaphthylene-7,10-dione using acenaphthene, aluminum chloride, sodium chloride and succinic anhydride by multistep process. The reported literature illustrates reaction of acenaphthene and succinic anhydride using AlCl₃ resulting in 80% product with two positional isomers. These isomers were isolated and converted into ethyl or methyl ester using respective alcohols and catalytic mineral acid. The ester of ester of β-(3-acenaphthoyl) propionic acid cyclized to synthesize 3,4,8,9- tetrahydrocyclohepta[fg]acenaphthylene-7,10-dione by using aluminum chloride and sodium chloride eutectic mixture by heating at high temperature resulting in 43% yield. The reaction scheme is as mentioned below:

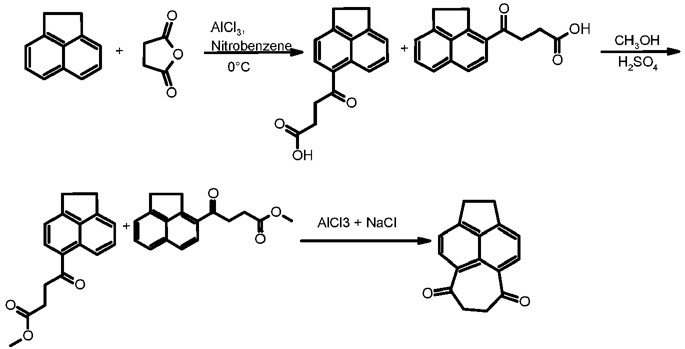

The synthesis of cyclic peri-succinoylacenaphthene and related 1,4-diketo compounds, especially 3,4,8,9-tetrahydrocyclohepta[fg]acenaphthylene-7,10-dione known in the art are therefore not a single step process and stringent requirements are required in terms of purification of intermediates and use of metal catalysts and solvents.

Another disadvantage of the prior art processes is their low yield. Hence, the methods described above are not commercially viable option for the synthesis of cyclic peri-succinoylacenaphthene and related 1,4-diketo compounds, especially 3,4,8,9-tetrahydrocyclohepta[fg]acenaphthylene-7,10-dione.

In order to overcome the problems associated with prior art, there is a need to develop an efficient and cost-effective method for the commercial scale production of cyclic peri-succinoylacenaphthene and related 1,4-diketo compounds (I), especially 3,4,8,9-tetrahydrocyclohepta[fg]acenaphthylene-7,10-dione (Ia).

### SUMMARY OF THE INVENTION

The main object of the present invention is therefore to provide a particularly advantageous simplified process for the synthesis of cyclic peri-succinoylacenaphthene and related 1,4-diketo compounds, especially 3,4,8,9-tetrahydrocyclohepta[fg]acenaphthylene-7,10-dione (Ia) with improved yield using less stringent metal catalysts and solvents.

The present invention therefore relates to a novel process for the preparation of a cyclic peri-succinoylacenaphthene and related 1,4-diketo compound of general formula (I) 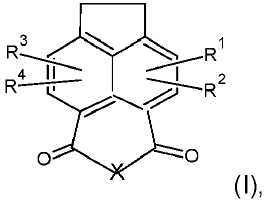 where
R¹, R², R³, and R⁴ are independently hydrogen, halogen, , -OR⁶,; in which R⁶ is C1-C6 alkyl, , (C6-C10)aryl-(C1-C6)alkyl, in which the alkyl and/or aryl radicals can be substituted by, C1-C6 alkoxy, or halogen, and X being a C2 alkylene or alkenyl group with possible substituents including alkyl groups, alkenyl groups, aralkyl groups or aralkenyl groups.
comprising:
(a) contacting an acenaphthene compound of general formula (II) 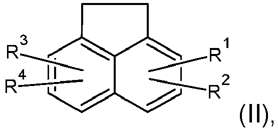 where
   R¹, R², R³, and R⁴ have the meanings already indicated, with a high boiling aprotic non-reactive solvent;
(b) adding cyclic acid anhydride of general formula (III) to step (a) forming a mixture; 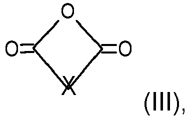 where, X being a C2 alkylene or alkenyl group with possible substituents including alkyl groups, alkenyl groups, aralkyl groups or aralkenyl groups,
(c) cooling the temperature of the mixture to -15°C to ambient temperature and adding a Lewis acid to said mixture; and
(d) increasing the temperature to 80°C to 150°C for 5 to 20 hours with further addition of the Lewis acid and
   wherein peri positions of the acenaphthene compound is free to react with the cyclic acid anhydride of general formula (III).

Preferred single pot process for the preparation of a cyclic peri-succinoylacenaphthene and related 1,4-diketo compound is 3,4,8,9-tetrahydrocyclohepta[fg]acenaphthylene-7,10-dione of formula (Ia) 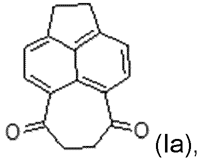 wherein R¹, R², R³ and R⁴ are all hydrogen, and X being CH₂-CH₂ group.

In particular, simple process for the conversion of an acenaphthene compound to a cyclic peri-succinoylacenaphthene and related 1,4-diketo compounds in better yield comprising:
(a) contacting the acenaphthene compound with a cyclic dicarboxylic acid anhydride e.g. succinic anhydride, maleic anhydride in a high boiling aprotic non-reactive solvent, wherein the acenaphthene compound reacts with the said cyclic dicarboxylic acid anhydride e.g. succinic anhydride, maleic anhydride or a cyclic dicarboxylic acid anhydride compound by a Friedel-Crafts reaction, at a temperature at about -15°C to ambient temperature, preferably 0°C to 15°C, more preferably at 0°C, followed by stirring at the same temperature until all the acenaphthene get completely consumed to a reaction mixture, and
(b) optimum proportion of the same Lewis acid is added to the reaction mixture at optimum conditions followed by heating at about 80°C to 150°C, preferably between 90°C to 120°C for about 5 to 20 hours;
wherein a peri position of the acenaphthene is free to react with the cyclic dicarboxylic acid anhydride e.g. succinic anhydride, maleic anhydride by the Friedel-Crafts reaction with the Lewis acid followed by high temperature cyclization reaction of in-situ developed gamma-keto acids of the acenaphthene compound to the peri-succinoylacenaphthene compound in a single pot reaction system by further addition of optimum proportion of the same Lewis acid. In yet another embodiment of the present invention, the cyclic dicarboxylic acid anhydrides chosen from succinic anhydride, maleic anhydride, phthalic anhydride, trimellitic anhydride, hexahydrophthalic anhydride, methyl hexahydrophthalic anhydride, methyl tetrahydrophthalic anhydride, tetrahydrophthalic anhydride, tetrachlorophthalic anhydride, tetrabromophthalic anhydride, dodecenylsuccinic anhydride, more preferably, the cyclic dicarboxylic acid anhydride comprises succinic anhydride, maleic anhydride.

In yet another embodiment of the present invention, the high boiling aprotic non-reactive solvents chosen from nitrobenzene, mono chlorobenzene, ortho dichlorobenzene or any other high boiling aprotic non-reactive solvents. The suitable solvents could be any non-reactive solvents used for Friedel-Crafts reaction.

In yet another embodiment of the present invention, the Lewis acid used in the reaction is an aluminum halide, a boron halide, a titanium halide, a tin halide, an iron halide, or an aluminum chloride, preferably an aluminum chloride.

In yet another embodiment of the present invention, the optimum amount of Lewis acid for the complete conversion of the acenaphthene compound for the initial reaction mixture is such that the Lewis acid/ acenaphthene compound molar ratio is from 1 to 3, preferably 2 to 2.5.

In yet another embodiment of the present invention, the optimum amount of Lewis acid for the high temperature cyclization reaction step to the cyclic peri-succinoylacenaphthene and related 1,4-diketo compound is such that the Lewis acid/ acenaphthene compound molar ratio is from 2 to 7, preferably 2.2 to 6.6.

In yet another embodiment of the present invention, the temperature for the complete conversion of the acenaphthene compound to the initial reaction mixture is between 0°C to ambient temperature, preferably 0°C to 15°C, more preferably at 0°C.

In yet another embodiment of the present invention, the optimum temperature for the high temperature cyclization reaction step to the cyclic peri-succinoylacenaphthene and related 1,4-diketo compound is between 80°C to 150°C, preferably between 90°C to 120°C.

In yet another embodiment of the present invention, the optimum time taken for the complete conversion of the acenaphthene compound to the initial reaction mixture is between 1 to 9 hours, preferably 5 to 8 hours.

In yet another embodiment of the present invention, the optimum time taken for the high temperature cyclization reaction step to the cyclic peri-succinoylacenaphthene and related 1,4-diketo compound is between 5 to 15 hours, preferably 7 to 12 hours. In one of the preferred embodiments of the present invention, current method involve reaction of acenaphthene (or substituted acenaphthene) with cyclic dicarboxylic acid anhydride e.g. succinic anhydride, maleic anhydride, where the peri position of acenaphthene (or substituted acenaphthene) is free to react with the said cyclic dicarboxylic acid anhydride e.g. succinic anhydride, maleic anhydride at -15°C to 0°C in solvents such as nitrobenzene, mono chlorobenzene, ortho dichlorobenzene or any other high boiling aprotic non-reactive solvents. Further, an optimum proportion of aluminum chloride was added at optimum reaction conditions and the reaction was further heated at 80°C to 150°C for 5 to 20 hours for the high temperature cyclization reaction to give desired peri-succinoylacenaphthene compound.

In yet another preferred embodiment of the present invention, current method involves purification steps, wherein upon cooling, reaction mixture was dumped in ice cold water and product isolated by steam distillation. Further, crude product was purified by high temperature alkali treatment to yield pure 3,4,8,9-tetrahydrocyclohepta[fg]acenaphthylene-7,10-dione (Ia) in high yield, wherein the yield of the desired cyclic peri-succinoylacenaphthene and related 1,4-diketo compound of general formula (I) is above 50%, preferably 50-90%, and more preferably 50-70%.

In yet another preferred embodiment of the present invention, current method involves purification step comprising by boiling with the cyclic peri-succinoylacenaphthene and related 1,4-diketo compound of general formula (I) with an alkali. The process according to the current method, wherein the alkali comprises sodium carbonate or sodium hydroxide solution.

In yet another embodiment of the present invention, the use of the compound (I) for synthesis of pigments and dye for high end applications, preferably as a colorant in synthetic polar polymers for fiber applications, electrophotographic toners, developers in powder coating materials, and ink-jet inks.

In yet another embodiment of the present invention, the use of the compound (I) for further synthesis of various intermediates for drugs or pharmaceutically active composition/ intermediates.

### DETAILED DESCRIPTION

Surprisingly it has been found that the cyclic peri-succinoylacenaphthene and related 1,4-diketo compounds can be prepared with ease and simplicity - in a single pot process wherein, an acenaphthene compounds can be transferred to a cyclic peri-succinoylacenaphthene and related 1,4-diketo compounds by using cyclic dicarboxylic acid anhydride e.g. succinic anhydride, maleic anhydride acylation with Lewis acid catalyzed Friedel Crafts reaction followed by a high temperature cyclization by using optimum proportion of same Lewis acid catalyst.

The improved process of the present invention is intended for use in any acenaphthene compounds as described in general formula (II), where R¹, R², R³, and R⁴ are independently hydrogen, halogen, -OR⁶; in which R⁶ is C1-C6 alkyl, (C6-C10)aryl-(C1-C6)alkyl, in which the alkyl and/or aryl radicals can be substituted by, C1-C6 alkoxy, or halogen, and X being a C2 alkylene or alkenyl group with possible substituents including alkyl groups, alkenyl groups, aralkyl groups or aralkenyl groups.

This simplified improved process required that peri positions of the acenaphthene compound is free to react with the cyclic dicarboxylic acid anhydride e.g. succinic anhydride, maleic anhydride via Friedel-Crafts acylation followed by high temperature cyclization in presence of Lewis acid in a high boiling aprotic non-reactive solvent, in a single pot reaction system.

In the process, acenaphthene of general formula (II) is acylated with cyclic dicarboxylic acid anhydride e.g. succinic anhydride, maleic anhydride with a Friedel-Crafts catalysts based on a Lewis acid or a mixture of Lewis acid in a high boiling aprotic non-reactive solvent for a time and temperature optimum to give an initial reaction mixture. This initial reaction mixture is then subjected to the high temperature cyclization with the addition of additional amount of same Lewis acid for a time and temperature optimum to produce a final reaction mixture comprising cyclic peri-succinoylacenaphthene and related 1,4-diketo compounds of general formula (I), wherein the single-pot process is resulted in better yield and uses of less stringent metal catalysts and solvents.

The entire reaction process of the current invention can be expressed by the following reaction formula: 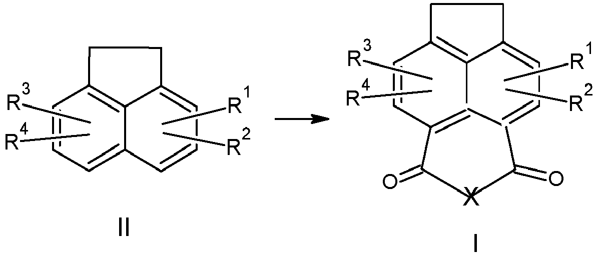 where R¹, R², R³, and R⁴ are independently hydrogen, halogen, , -OR⁶; in which R⁶ is, C1-C6 alkyl, (C6-C10)aryl-(C1-C6)alkyl, in which the alkyl and/or aryl radicals can be substituted by C1-C6 alkoxy, or halogen, and X being a C2 alkylene or alkenyl group with possible substituents including alkyl groups, alkenyl groups, aralkyl groups or aralkenyl groups.

The cyclic acid anhydride according to the present invention is a compound of the general formula depicted below: 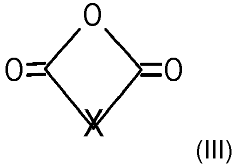 with X being an, optionally substituted C2 alkylene or alkenyl group. Possible substituents on the C2 alkylene or alkenyl group include alkyl groups, alkenyl groups, aralkyl groups or aralkenyl groups. The cyclic anhydride of a saturated or unsaturated dicarboxylic acid is preferably selected from the group comprising of succinic anhydride, maleic anhydride, phthalic anhydride, trimellitic anhydride, hexahydrophthalic anhydride, methyl hexahydrophthalic anhydride, methyl tetrahydrophthalic anhydride, tetrahydrophthalic anhydride, tetrachlorophthalic anhydride, tetrabromophthalic anhydride, dodecenylsuccinic anhydride. More preferably, the cyclic acid anhydride of general formula (III) comprising of succinic anhydride, maleic anhydride or a cyclic dicarboxylic acid anhydride..

Most preferably it is succinic anhydride or maleic anhydride.

The Lewis acid catalysts are characterized by the presence of a vacant orbital which can accept an available electron pair, either unshared, e.g. on an oxygen, sulfur, or halide atom, or in a π orbital, of a Lewis base type compound to form a covalent bond. Exemplary of suitable Lewis acid catalysts are aluminum chloride, stannic chloride, stannic bromide, zinc chloride, zinc bromide, antimony pentachloride, titanium tetrachloride, ferric chloride, gallium trichloride, zirconium tetrachloride, mercuric chloride, chromium trichloride and like metal halide agents exhibiting Friedel-Crafts type catalytic activity. Preferred of such catalysts are aluminum halide, boron halide, titanium halide, tin halide, iron halide, or aluminum chloride. Aluminum chloride is especially preferred. Mixtures of these catalysts can also be used.

The Lewis acid catalyst employed for initial reaction step, wherein cyclic dicarboxylic acid anhydride e.g. succinic anhydride, maleic anhydride is acylated to acenaphthene compounds is such that the Lewis acid/acenaphthene compound molar ration is from 1 to 3, preferably 2 to 2.5. On the other hand, optimum proportion of same Lewis acid catalyst employed for the final high temperature reaction step is such that the Lewis acid/ acenaphthene compound molar ration is from 2 to 7, preferably 2.2 to 6.6.

The solvent used in the present invention comprises of one or more kinds of high boiling aprotic non-reactive solvents selected from the group consisting of nitrobenzene, mono chlorobenzene, ortho dichlorobenzene or any other high boiling aprotic non-reactive solvents. A preferred organic solvent used in the present invention consists of one or more kinds of organic solvents selected from the group consisting of nitrobenzene, mono chlorobenzene, and ortho dichlorobenzene.

Advantageously, excess amount of aprotic non-reactive solvents is used for the reaction. Preferably around 5 folds or more of excess amounts of nitrobenzene, mono chlorobenzene, ortho dichlorobenzene or any other high boiling aprotic non-reactive solvents. For example, from 5 to 30 moles, preferably 5 to 15 moles of nitrobenzene, or mono chlorobenzene, ortho dichlorobenzene can be used per mole of acenaphthene. Important to note that the alternate option with lesser amount of aprotic non-reactive solvents is also possible and are part of this invention.

The initial reaction, wherein Friedel-Crafts acylation reaction is carried out at a temperature between -15°C and 15°C and preferably between 0° to 5°C, and in an inert organic solvent. The reaction is advantageously carried out by reacting the starting materials at from -15°C and 15°C, preferably from 0° to 5°C, in particular at 0°C, under atmospheric pressure and continued for 1 to 9 hours, preferably 5 to 8 hours.

The Friedel-Crafts reaction is advantageously carried out in the manner, for example:
(a) contacting an acenaphthene compound of general formula (II) 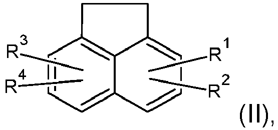
   where R¹, R², R³, and R⁴ are independently hydrogen, halogen, -OR⁶; in which R⁶ is, C₁-C₆ alkyl, (C₆-C₁₀)aryl-(C₁-C₆)alkyl, in which the alkyl and/or aryl radicals can be substituted by, C₁-C₆ alkoxy, or halogen,
   with a high boiling aprotic non-reactive solvent;
(b) adding cyclic acid anhydride of general formula (III) to step (a) forming a mixture; 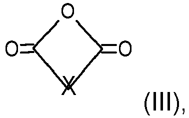 where, X being a C2 alkylene or alkenyl group with possible substituents including alkyl groups, alkenyl groups, aralkyl groups or aralkenyl groups, and cooling the temperature of the mixture at from -15°C to 5°C, preferably from 0° to 5°C, in particular at 0°C and adding a Lewis acid in portions, while maintaining the temperature at 0°C and
(c) the reaction mixture was stirred for another 1 to 9 hours, preferably 5 to 8 hours at the same temperature until all acenaphthene get completely consumed.

Advantageously, the acenaphthene compound of general formula (II) is initially taken together with the solvent while maintaining the anhydrous inert condition by using dry nitrogen gas and then the cyclic dicarboxylic acid anhydride e.g. succinic anhydride, maleic anhydride is added, although the converse sequence is also possible.

The final reaction, wherein Lewis acid catalyzed cyclization reaction is carried out at a temperature between 80°C and 150°C and preferably between 90°C to 120°C, and in an inert organic solvent. The reaction is advantageously carried out by slowly raising the temperature up to between 80°C and 150°C and preferably between 90°C to 120°C, under atmospheric pressure and continued for 5 to 20 hours, preferably 7 to 12 hours.

The final reaction is the high temperature cyclization reaction to produce a final reaction mixture comprising cyclic peri-succinoylacenaphthene and related 1,4-diketo compound of general formula (I) 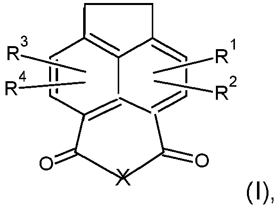
in which, R¹, R², R³, R⁴ and X have the meanings already indicated,
wherein the single pot process is resulted in better yield using less stringent metal catalysts, solvents, and eliminate all intermediate isolation.

The final high temperature cyclization reaction is advantageously carried out in the following manner, for example:
(a) further optimum proportion of same Lewis acid was added carefully to the initial reaction mixture while maintaining an optimum reaction condition;
(b) maintaining the temperature to 80°C to 150°C with optimum proportion of Lewis acid for 5 to 20 hours; and
(c) and continued for 5 to 20 hours, preferably 7 to 12 hours until all in-situ developed gamma-keto acids get completely consumed.

As noted above, the process is carried out under substantially anhydrous conditions. Trace amounts of water are tolerable; however, it is desirable to maintain the reaction mixture in the process as dry as possible.

Working up of the reaction mixture and isolation of the crude product is generally carried out in a conventional manner by pouring the final reaction mixture into water and/ or ice, and then steam distillation of aprotic non-reactive organic solvent phase.

The crude product is advantageously washed neutral with alkali treatment, preferably by boiling in dilute sodium carbonate or sodium hydroxide solution and then with water. The solvent is removed, and the final product then isolated.

This procedure results in not only lower solvent consumption but also less environmental pollution due to single pot synthesis process - so purification of intermediates and use of excess metal catalysts and solvents are avoided.

The methods described herein will now be discussed with specific reference to various examples. These examples are presented to further explain the invention and are not intended, or to be taken, to limit the scope of the invention and are rather provided as exemplary embodiments - the scope of the invention is as defined by the claims appended to this description. Unless indicated otherwise, parts are parts by weight, temperature is °C or is at ambient temperature, and pressure is at or near atmospheric.

### EXAMPLE 1:

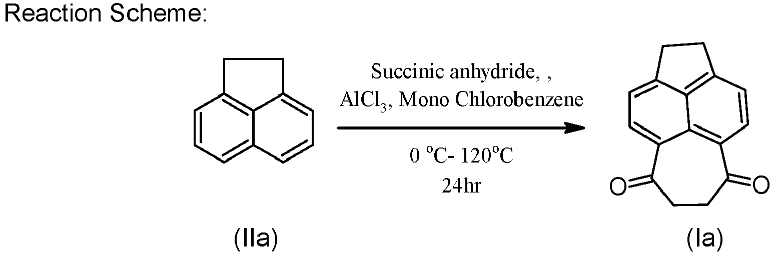

In 500 mL four neck round-bottom flask (RBF), acenaphthene (1eq) was taken to which slowly chloro-benzene (20 fold) was added maintaining the inert condition using dry nitrogen gas followed by addition of Succinic anhydride (1.1 eq). This mixture was cooled to 0°C and slowly aluminum chloride (2.2 eq) was introduced in portion over the period of 1-2 hours maintaining the temperature at 0°C. After completion of addition, reaction mixture was stirred for 6 hours at same temperature until all acenaphthene get completely consumed. Reaction was monitored by thin-layer chromatography (TLC) and high-performance liquid chromatography (HPLC) which shown the formation of β-(3-acenaphthoyl) propionic acid and β-(1-acenaphthoyl) propionic acid. Remaining portion of aluminum chloride (4.4 eq) was added carefully to reaction mixture at same temperature. Slowly temperature of reaction was increased up to 90°C and continued for next 15-18 hours. Reaction was monitored by TLC and HPLC. After complete consumption of β-(3-acenaphthoyl) propionic acid, reaction was stopped and cooled to room temperature and quenched by 150 ml ice cold water. Chlorobenzene was removed by steam distillation. Brown product was subjected to purification by boiling in Na₂CO₃ solution to yield 50% 3,4,8,9- tetrahydrocyclohepta[fg]acenaphthylene-7,10-dione.

### EXAMPLE 2:

Acenaphthene (1 eq) was taken in 500 mL four neck RBF equipped with overhead stirrer, refluxing condenser and thermometer to which ortho dichlorobenzene (ODCB) (8 fold) was added maintaining the inert condition using nitrogen followed by addition of Succinic anhydride (1.1 eq). This mixture was cooled to 0°C-5°C and slowly aluminum chloride (2.2 eq) was introduced in portion within 1-2 hours maintaining the temperature at 0°C. After completion of addition, reaction mixture was stirred for 6-7 hours at 0°C until all acenaphthene get completely consumed. Reaction was monitored by TLC and HPLC which shown the formation of β (acenaphthoyl)propionic acid. Second portion of aluminum chloride (4.4 eq) was added carefully to reaction mixture at 0°C. Slowly temperature of reaction was increased up to 90°C-95°C and continued for next 16-18 hours. Reaction progress was monitored by HPLC. After complete consumption of β-(3-acenaphthoyl) propionic acid, reaction was stopped and cooled to room temperature and quenched by 150 ml ice cold water. ortho dichlorobenzene (ODCB) was removed by steam distillation. Product was purified by boiling in NaOH solution to yield 54% 3,4,8,9-tetrahydrocyclohepta[fg]acenaphthylene-7,10-dione.

### EXAMPLE 3:

In 500 mL four neck RBF acenaphthene (1eq) was taken to which slowly ortho dichlorobenzene (20 fold) was added maintaining the inert condition using nitrogen followed by addition of succinic anhydride (1.1 eq). This mixture was cooled to 0°C and slowly aluminum chloride (2.2 eq) was introduced in portion over the period of 1-2 hours maintaining the temperature at 0°C. After completion of addition, reaction mixture was stirred for 6-7 hours at 0°C until all acenaphthene get completely consumed. Reaction was monitored by TLC and HPLC which shown the formation of β-(3-acenaphthoyl) propionic acid and β-(1-acenaphthoyl) propionic acid. Second lot of aluminum chloride (6.6 eq) was added carefully to reaction mixture at 0°C. Slowly temperature of reaction was increased up to 90°C-100°C and continued for next 16-18 hours. Reaction was monitored HPLC. After complete consumption of β-(3-acenaphthoyl) propionic acid, reaction was stopped and cooled to room temperature and quenched by 200 mL ice cold water. ODCB was removed by steam. Desired product was filtered and subjected to alkali purification to yield 52% 3,4,8,9-tetrahydrocyclohepta[fg]acenaphthylene-7,10-dione.

### EXAMPLE 4:

Acenaphthene (1eq) and ortho dichlorobenzene (20 fold) was taken in 500 mL four neck RBF equipped with overhead stirrer, refluxing condenser and thermometer maintaining the inert condition using nitrogen followed by addition of succinic anhydride (1.1 eq) This mixture was cooled to 0°C and stirred for 5 min at same temperature. Slowly aluminum chloride (2.2 eq) was introduced in portion over the period of 1-2 h maintaining the temperature at 0°C. After completion of addition, reaction mixture was stirred for 6-7 hr at same temperature until all acenaphthene get completely consumed. Reaction was monitored by TLC and HPLC which shown the formation of β-(acenaphthoyl)propionic acid. Second portion of aluminum chloride (4.4eq) was added carefully to reaction mixture at same temperature. Slowly temperature of reaction was increased up to 115-120°C and continued for next 17-18 hours. After complete consumption of β-(3-acenaphthoyl) propionic acid, reaction was stopped and cooled to room temperature and quenched by 200 mL ice cold water. ODCB was removed by steam. Product was filtered and purified by boiling in Na₂CO₃ solution to yield 55% 3,4,8,9-tetrahydrocyclohepta[fg]acenaphthylene-7,10-dione.

### ANALYSIS:

The cyclic peri-succinoylacenaphthene and related 1,4-diketo compounds, especially (Ia) is fully characterized by ¹HNMR and Mass Spectroscopy and data are presented in Table 1:

**Table 1**

| Compound | ¹H-NMR (DMSO-d6, 400Hz) | Mass (XXX) |
|---|---|---|
| 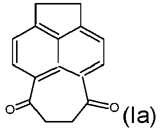 | 8.41(d, J= 7.4Hz, 2H), 7.57 (d, J= 7.52Hz, 2H), 3.44 (s, 4H), 3.05 (s, 4H) | 237(M+H)⁺ |

The cyclic peri-succinoylacenaphthene and related 1,4-diketo compounds thus obtained serves as a valuable starting material for the preparation of dyes and dye intermediates. Most importantly, the intermediate 1, 4, 5, 8-naphtalene tetracarboxylic acid of the formula (IVa), and various derivatives (IV) are suitable for synthesis of pigments and dye for high end applications, preferably as a colorant in synthetic polar polymers for fiber applications, electrophotographic toners, developers in powder coating materials, and ink-jet inks.

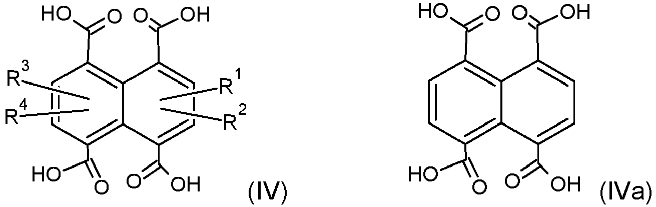

Similarly, the cyclic peri-succinoylacenaphthene and related 1,4-diketo compounds thus obtained serves as a valuable starting material for the preparation of various intermediates for drugs or pharmaceutically active composition/ intermediates.

## Claims

1. A process for preparing a cyclic peri-succinoylacenaphthene and related 1,4-diketo compound of general formula (I) 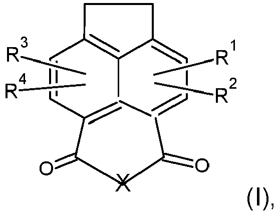 where
R¹, R², R³, and R⁴ are independently hydrogen, halogen, or -OR⁶; in which R⁶ is C1-C6 alkyl, , (C6-C10)aryl-(C1-C6)alkyl, in which the alkyl and/or aryl radicals can be substituted by C1-C6 alkoxy, or halogen, and
X being a C2 alkylene or alkenyl group with possible substituents including alkyl groups, alkenyl groups, aralkyl groups or aralkenyl groups,
comprising:
a) contacting an acenaphthene compound of general formula (II) 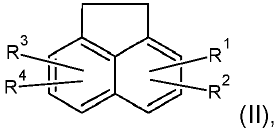
where, R¹, R², R³, and R⁴ have the meanings already indicated,
with a high boiling aprotic non-reactive solvent;
b) adding cyclic acid anhydride of general formula (III) to step (a) forming a mixture; 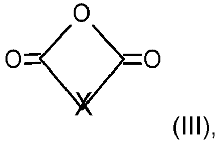 where
X being a C2 alkylene or alkenyl group with possible substituents including alkyl groups, alkenyl groups, aralkyl groups or aralkenyl groups,
c) cooling the mixture to -15°C to ambient temperature and adding a Lewis acid to said mixture; and
d) increasing the temperature to 80°C to 150°C for 5 to 20 hours with further addition of the Lewis acid;
wherein peri positions of the acenaphthene compound is free to react with the cyclic acid anhydride (III).

2. The process according to claim 1, further comprising steps of:
cooling the mixture of step (d) to room temperature followed by quenching with water;
removing the high boiling aprotic non-reactive solvent by steam distillation; and
recovering the peri-succinoylacenaphthene compound of general formula (I).

3. The process according to claims 1 to 2, wherein further comprising purification by boiling the cyclic peri-succinoylacenaphthene and related 1,4-diketo compound of general formula (I) with an alkali.

4. The process according to claim 3, wherein the alkali comprises sodium carbonate or sodium hydroxide solution.

5. The process according to claim 1, wherein the cyclic acid anhydride of general formula (III), comprises succinic anhydride, maleic anhydride, phthalic anhydride, trimellitic anhydride, hexahydrophthalic anhydride, methyl hexahydrophthalic anhydride, methyl tetrahydrophthalic anhydride, tetrahydrophthalic anhydride, tetrachlorophthalic anhydride, tetrabromophthalic anhydride, dodecenylsuccinic anhydride.

6. The process according to claim 5, wherein the cyclic acid anhydride of general formula (III), preferably comprises succinic anhydride, maleic anhydride.

7. The process according to claims 1 to 3, wherein the said cyclic peri-succinoylacenaphthene and related 1,4-diketo compound is 3,4,8,9-tetrahydrocyclohepta[fg]acenaphthylene-7,10-dione of formula (Ia)

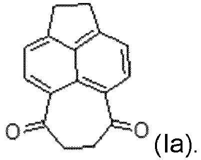

8. The process according to claim 1 to 2, wherein the said acenaphthene compound is 1,2-dihydroacenaphthylene of formula (IIa)

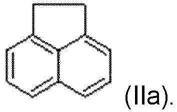

9. The process according to claim 1, wherein the high boiling aprotic non-reactive solvents chosen from nitrobenzene, mono chlorobenzene, ortho dichlorobenzene or any other high boiling aprotic non-reactive solvents.

10. The process according to claim 1, wherein the Lewis acid is selected from the group comprising of aluminum halide, boron halide, titanium halide, tin halide or iron halide, preferably aluminum chloride.

11. The process according to claim 1, wherein the Lewis acid/ acenaphthene compound in step (c) is in the ration from 1 to 3, preferably from 2 to 2.5.

12. The process according to claim 1, wherein step (c) comprises cooling preferably from 0°C to about 15°C, more preferably at 0°C.

13. The process according to claim 1, wherein time taken for the complete conversion of the acenaphthene compound for the step (c) is between 1 to 9 hours, preferably between 5 to 8 hours.

14. The process according to claim 1, wherein the Lewis acid/ acenaphthene compound in step (d) is from 2 to 7, preferably from 2.2 to 6.6.

15. The process according to claim 1, wherein step (d) comprises increasing the temperature preferably between 90°C to 120°C.

16. The process according to claim 1, wherein time taken for step (d) preferably between 7 to 12 hours.

## Patentansprüche

1. Verfahren zur Herstellung eines cyclischen peri-Succinoyl-Acenaphthens und verwandter 1,4-Diketo-Verbindung der allgemeinen Formel (I) 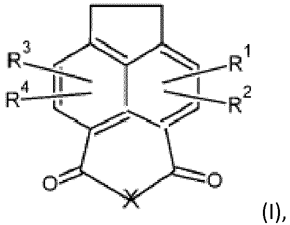 wobei
R¹, R², R³ und R⁴ unabhängig Wasserstoff, Halogen, oder -OR⁶ sind; worin R⁶ C1- C6-Alkyl,, (C6-C10)-Aryl-(C1-C6)-Alkyl, worin die Alkyl und/oder Arylradikale durch C1-C6-Alkoxy oder Halogen substituiert sein können, ist, und
X eine C2-Alkylen- oder Alkenylgruppe mit möglichen Substituenten, einschließlich Alkylgruppen, Alkenylgruppen, Aralkylgruppen oder Aralkenylgruppen, ist, umfassend:
a) Inkontaktbringen einer Acenaphthen-Verbindung der allgemeinen Formel (II), 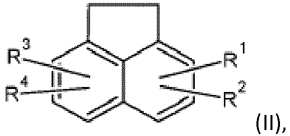 wobei R¹, R², R³ und R⁴ die bereits angegebenen Bedeutungen haben, mit einem hochsiedenden, aprotischen, nicht reaktiven Lösungsmittel;
b) Zugeben cyclischen Säureanhydrids der allgemeinen Formel (III) zu Schritt (a) unter Bildung einer Mischung; 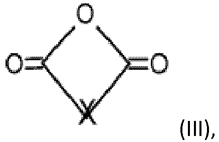 wobei
X eine C2-Alkylen- oder Alkenylgruppe mit möglichen Substituenten, einschließlich Alkylgruppen, Alkenylgruppen, Aralkylgruppen oder Aralkenylgruppen, ist,
c) Kühlen der Mischung auf -15 °C bis Umgebungstemperatur und Zugabe einer Lewis-Säure zu der Mischung; und
d) Erhöhen der Temperatur auf 80 °C bis 150 °C für 5 bis 20 Stunden mit weiterer Zugabe der Lewis-Säure;
wobei peri-Positionen der Acenaphthen-Verbindung frei ist, um mit dem cyclischen Säureanhydrid (III) zu reagieren.

2. Verfahren gemäß Anspruch 1, ferner umfassend die Schritte:
Kühlen der Mischung aus Schritt (d) auf Raumtemperatur, gefolgt von Quenchen mit Wasser;
Entfernen des hochsiedenden, aprotischen, nicht reaktiven Lösungsmittels durch Dampfdestillation; und
Rückgewinnen der peri-Succinoyl-Acenaphthen-Verbindung der allgemeinen Formel (I).

3. Verfahren gemäß Ansprüche 1 bis 2, wobei ferner die Reinigung durch Sieden des cyclischen peri-Succinoyl-Acenaphthens und verwandter 1,4-Diketo-Verbindung der allgemeinen Formel (I) mit Alkali umfasst ist.

4. Verfahren gemäß Anspruch 3, wobei das Alkali Natriumcarbonat- oder Natriumhydroxidlösung umfasst.

5. Verfahren gemäß Anspruch 1, wobei das cyclische Säureanhydrid der allgemeinen Formel (III) Bernsteinsäureanhydrid, Maleinsäureanhydrid, Phthalsäureanhydrid, Trimellitsäureanhydrid, Hexahydrophthalsäureanhydrid, Methylhexahydrophthalsäureanhydrid, Methyltetrahydrophthalsäureanhydrid, Tetrahydrophthalsäureanhydrid, Tetrachlorphthalsäureanhydrid, Tetrabromphthalsäureanhydrid, Dodecenylbernsteinsäureanhydrid umfasst.

6. Verfahren gemäß Anspruch 5, wobei das cyclische Säureanhydrid der allgemeinen Formel (III) bevorzugt Bernsteinsäureanhydrid, Maleinsäureanhydrid umfasst.

7. Verfahren gemäß den Ansprüchen 1 bis 3, wobei das cyclische peri-Succinoyl-Acenaphthen und verwandte 1,4-Diketo-Verbindung 3,4,8,9-Tetrahydrocyclohepta[fg]acenaphthylen-7,10-dion der Formel (Ia) ist

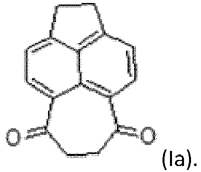

8. Verfahren gemäß Anspruch 1 bis 2, wobei die Acenaphthen-Verbindung 1,2-Dihydroacenaphthylen der Formel (IIa) ist

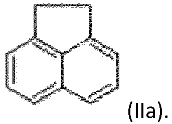

9. Verfahren gemäß Anspruch 1, wobei die hochsiedenden, aprotischen, nicht reaktiven Lösungsmittel ausgewählt sind aus Nitrobenzol, Monochlorbenzol, ortho-Dichlorbenzol oder beliebigen anderen hochsiedenden, aprotischen, nicht reaktiven Lösungsmitteln.

10. Verfahren gemäß Anspruch 1, wobei die Lewis-Säure ausgewählt ist aus der Gruppe, umfassend Aluminiumhalogenid, Borhalogenid, Titanhalogenid, Zinnhalogenid oder Eisenhalogenid, bevorzugt Aluminiumchlorid.

11. Verfahren gemäß Anspruch 1, wobei die Lewis-Säure/Acenaphthen-Verbindung in Schritt (c) im Verhältnis von 1 bis 3, bevorzugt von 2 bis 2,5, vorliegt.

12. Verfahren gemäß Anspruch 1, wobei Schritt (c) ein Kühlen bevorzugt von 0 °C bis etwa 15 °C, besonders bevorzugt bei 0°C, umfasst.

13. Verfahren gemäß Anspruch 1, wobei die Zeitdauer für die vollständige Umwandlung der Acenaphthen-Verbindung für den Schritt (c) zwischen 1 und 9 Stunden, bevorzugt zwischen 5 und 8 Stunden, beträgt.

14. Verfahren gemäß Anspruch 1, wobei die Lewis-Säure/Acenaphthen-Verbindung in Schritt (d) von 2 bis 7, bevorzugt von 2,2 bis 6,6, beträgt.

15. Verfahren gemäß Anspruch 1, wobei Schritt (d) das Erhöhen der Temperatur bevorzugt zwischen 90 °C und 120 °C umfasst.

16. Verfahren gemäß Anspruch 1, wobei die Zeitdauer für Schritt (d) bevorzugt zwischen 7 und 12 Stunden beträgt.

## Revendications

1. Procédé de préparation d'un peri-succinoylacénaphtène cyclique et d'un composé 1,4-dicéto apparenté de formule générale (I) 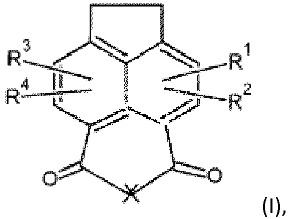 où
R¹, R², R³ et R⁴ sont indépendamment un atome d'hydrogène, un atome d'halogène ou -OR⁶; dans lequel R⁶ est un alkyle en C1-C6, un aryl(C6-C10)-alkyle(C1-C6), dans lequel les radicaux alkyle et/ou aryle peuvent être substitués par un alcoxy en C1-C6 ou un atome d'halogène, et
X est un groupe alkylène ou alcényle en C2 avec des substituants possibles, y compris des groupes alkyle, des groupes alcényle, des groupes aralkyle ou des groupes aralcényle, comprenant:
a) mettre en contact d'un composé acénaphtène de formule générale (II), 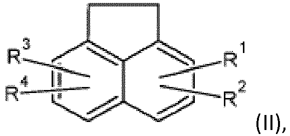 où R¹, R², R³ et R⁴ ont les significations déjà indiquées, avec un solvant aprotique, non réactif, à haut point d'ébullition;
b) ajouter d'un anhydride d'acide cyclique de formule générale (III) à l'étape (a) pour former un mélange ; 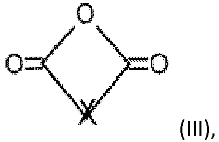 où
X est un groupe alkylène ou alcényle en C2 avec des substituants possibles, y compris des groupes alkyle, des groupes alcényle, des groupes aralkyle ou des groupes aralcényle,
c) refroidir le mélange à -15 °C à la température ambiante et ajouter un acide de Lewis audit mélange; et
d) augmenter la température de 80 °C à 150 °C pendant 5 à 20 heures avec l'addition supplémentaire de l'acide de Lewis ;
où les positions peri du composé acénaphtène sont libres de réagir avec l'anhydride d'acide cyclique (III).

2. Procédé selon la revendication 1, comprenant en outre les étapes:
refroidir le mélange de l'étape (d) à la température ambiante suivi par refroidissement rapide avec de l'eau;
éliminer le solvant aprotique, non réactif, à haut point d'ébullition par distillation à la vapeur; et
récupérer le composé peri-succinoylacénaphtène de formule générale (I).

3. Procédé selon les revendications 1 à 2, comprenant en outre la purification par ébullition du peri-succinoylacénaphtène cyclique et du composé 1,4-dicéto apparenté de formule générale (I) avec un alcali.

4. Procédé selon la revendication 3, dans lequel l'alcali comprend d'une solution du carbonate de sodium ou d'hydroxyde de sodium.

5. Procédé selon la revendication 1, dans lequel l'anhydride d'acide cyclique de formule générale (III) comprend l'anhydride succinique, l'anhydride maléique, l'anhydride phtalique, l'anhydride trimellitique, l'anhydride hexahydrophtalique, l'anhydride méthyl hexahydrophtalique, l'anhydride méthyl tétrahydrophtalique, l'anhydride tétrahydrophtalique, l'anhydride tétrachlorophtalique, l'anhydride tétrabromophtalique, l'anhydride dodécénylsuccinique.

6. Procédé selon la revendication 5, dans lequel l'anhydride d'acide cyclique de formule générale (III) comprend de préférence l'anhydride succinique, l'anhydride maléique.

7. Procédé selon les revendications 1 à 3, dans lequel ledit peri-succinoylacénaphtène cyclique et composé 1,4-dicéto apparenté est la 3,4,8,9-tétrahydrocyclohepta[fg]acénaphtylène-7,10-dione de formule (la)

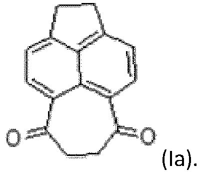

8. Procédé selon la revendication 1 à 2, dans lequel ledit composé acénaphtène est le 1,2-dihydroacénaphtylène de formule (IIa)

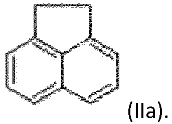

9. Procédé selon la revendication 1, dans lequel les solvants aprotiques, non réactifs, à haut point d'ébullition sont choisis parmi le nitrobenzène, le mono chlorobenzène, l'ortho dichlorobenzène ou tout autre solvant aprotique, non réactif, à haut point d'ébullition.

10. Procédé selon la revendication 1, dans lequel l'acide de Lewis est choisi dans le groupe comprenant l'halogénure d'aluminium, l'halogénure de bore, l'halogénure de titane, l'halogénure d'étain ou l'halogénure de fer, de préférence le chlorure d'aluminium.

11. Procédé selon la revendication 1, dans lequel le composé acide de Lewis/acénaphtène dans l'étape (c) est présent dans le rapport de 1 à 3, de préférence de 2 à 2,5.

12. Procédé selon la revendication 1, dans lequel l'étape (c) comprend le refroidir, de préférence de 0 °C à environ 15 °C, de préférence encore à 0 °C.

13. Procédé selon la revendication 1, dans lequel le temps pris pour la conversion complète du composé acénaphtène pour l'étape (c) est compris entre 1 à 9 heures, de préférence entre 5 à 8 heures.

14. Procédé selon la revendication 1, dans lequel le composé acide de Lewis/acénaphtène de l'étape (d) est de 2 à 7, de préférence de 2,2 à 6,6.

15. Procédé selon la revendication 1, dans lequel l'étape (d) comprend à augmenter la température, de préférence entre 90 °C et 120 °C.

16. Procédé selon la revendication 1, dans lequel le temps pris pour l'étape (d) est de préférence entre 7 et 12 heures.
